(19) Europäisches Patentamt
European Patent Office
Office européen des brevets

(11) Veröffentlichungsnummer: **0 307 676**
**A1**

(12) **EUROPÄISCHE PATENTANMELDUNG**

(21) Anmeldenummer: **88113834.1**

(51) Int. Cl.4: **C07D 231/14**

(22) Anmeldetag: **25.08.88**

(30) Priorität: **04.09.87 DE 3729598**

(43) Veröffentlichungstag der Anmeldung:
**22.03.89 Patentblatt 89/12**

(84) Benannte Vertragsstaaten:
**AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Sohn, Erich, Dr.**
**Troppauer, Strasse 14**
**D-8906 Gersthofen(DE)**
Erfinder: **Kunstmann, Rudolf, Dr.**
**Schlesierstrasse 9**
**D-8901 Aystetten(DE)**

(54) **Verfahren zur Herstellung von 1-Aryl-pyrazol-3-carbonsäureestern.**

(57) Verfahren zur Herstellung von Verbindungen der Formel I

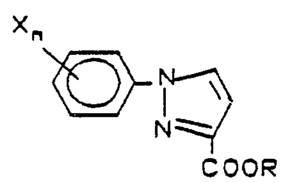

(I)

worin X = Halogen, (Halogen)alkyl oder(Halogen) alkoxy ; R = H oder ($C_1$-$C_6$)-Alkyl und n = 1 bis 5 bedeutet, wobei man ein Chlorhydrazon der Formel II

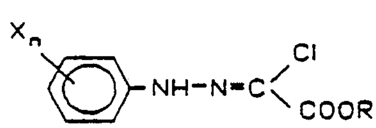

(II)

mit Norbornadien zwischen 0° und 200° C gegebenenfalls in Gegenwart eines Lösungsmittels und einer Hilfsbase umsetzt.

EP 0 307 676 A1

## Verfahren zur Herstellung von 1-Aryl-pyrazol-3-carbonsäureestern

1-Aryl-pyrazol-3-carbonsäureester der unten angegebenen Formel I sind hochwirksame Safener für Herbizide (DE-A 3 633 840). Die dort beschriebene Synthese dieser Pyrazole erfolgt durch Ringschlußreaktion von Phenylhydrazinen mit 4-Ethoxy-2-oxo-but-3-ensäureestern. Dabei fällt der gewünschte 3-Pyrazolcarbonsäureester neben dem entsprechenden 5-Pyrazolcarbonsäureester im Gemisch an. Eine aufwendige Isomerentrennung wird dadurch notwendig; anschließend muß das unerwünschte Nebenprodukt entsorgt werden. Das Verfahren weist also erhebliche technische Nachteile auf.

Es ist ferner bekannt, daß Pyrazole durch 1,3-dipolare Cycloadditionen zugänglich sind. So lassen sich Phenylsydnone mit Acetylenen zu Pyrazolen umsetzen (DE-A 1 261 125). Auch bei dieser Reaktion entstehen jedoch Isomerengemische (von 3- und 4-Pyrazolcarbonsäureestern).

Die selektive Synthese von 3-Pyrazolcarbonsäureestern ist nur auf folgendem umständlichen mehrstufigen Syntheseweg beschrieben:N-Phenyl-C-Acetyl-Nitrilimin wird mit Norbornadien zum Pyrazolinonorbornen cyclokondendiert, anschließend zum 3-Acetylpyrazol aromatisiert und mit $J_2$-NaOH zur Säure abgebaut [R. Huisgem et al., Chem. Ber. 101, 536 bis 551, (1968)]. Die Veresterung führt schließlich zum gewünschten Produkt.

Die Nachteile, die mit der Durchführung der bekannten Verfahren verbunden sind, werden durch das erfindungsgemäße Verfahren zur Herstellung von 3-Pyrazolcarbonsäureestern überwunden.

Überraschenderweise wurde gefunden, daß bei der Umsetzung von N-Phenyl-C-Alkoxycarbonyl-nitriliminen mit Norbornadien die Verbindungen der Formel I selektiv und in hohen Ausbeuten erhalten werden.

Gegenstand der vorliegenden Anmeldung ist daher ein Verfahren zur Herstellung von 1-Aryl-pyrazol-3-carbonsäureestern der Formel I

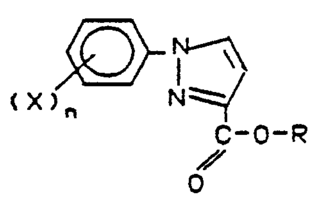

(I),

worin

X unabhängig voneinander Halogen, ($C_1$-$C_5$)-Alkyl, ($C_1$-$C_6$)-Alkoxy, Halogen($C_1$-$C_6$)alkyl oder Halogen($C_1$-$C_6$)alkoxy,

R H, ($C_1$-$C_6$)-Alkyl und

n eine ganze Zahl von 1 bis 5 bedeuten,

dadurch gekennzeichnet, daß man ein Chlorhydrazon der Formel II

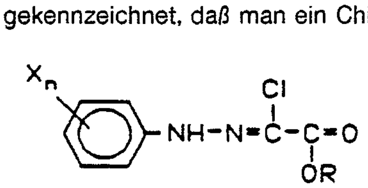

(II)

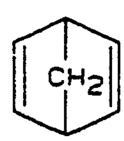

(III)

mit Norbornadien der Formel III umsetzt.

Die Umsetzung von Phenyl-alkoxycarbonyl-nitriliminen mit Verbindungen, die elektronenreiche Doppelbindungen besitzen war bereits bekannt: Umsetzung mit cyclischen Enaminen (Fusco et al. Gazz. Chim. Ital. 91 (1961) 1233 ff.) oder mit Bismorpholinethan-Verbindungen (Synthesis 1976 684/685). Symmetrische Ethen-Verbindungen lieferten hierbei nur geringe Ausbeuten an gewünschten Produkt.

Es war daher auch nicht zu erwarten, daß N-Phenyl-C-Alkoxycarbonyl-nitrilimine mit Verbindungen, die wie das Norbornadien unpolarisierte, elektronenarme Doppelbindungen besitzen, in hervorragenden Ausbeuten reagieren.

Das erfindungsgemäße Verfahren findet vorzugsweise Anwendung zur Herstellung von Verbindungen der Formel I worin R ($C_1$-$C_4$)-Alkyl, X = Halogen, $C_1$-Halogenalkyl oder $C_1$-Halogenalkoxy und n = 1 bis 3 bedeutet.

Unter Halogenalkyl- oder Halogenalkoxyresten sind insbesondere die Reste -$CF_3$ und -$OCF_3$ zu

verstehen.

Die Komponenten der Formeln II und III lassen sich ohne Lösungsmittel, vorzugsweise jedoch in einem inerten organischen Lösungsmittel wie z.B. Benzol, Toluol, Xylol, Chlorbenzol, Mono-, Di- oder Triethylenglykolether, Dimethylformamid, Dimethylsulfoxid, N-Methylpyrrolidon oder Pyridin umsetzen.

Das Verfahen wird vorteilhaft in Gegenwart einer organischen oder anorganischen Hilfsbase wie beispielsweise einem Trialkylamin, Pyridin, einem Alkalicarbonat oder -hydrogencarbonat, Erdalkalicarbonat oder -hydrogencarbonat, Alkalihydroxyd oder Alkalialkoholat durchgeführt.

Die Base wird zweckmäßigerweise in Überschuß eingesetzt, vorteilhaft in einer bis zu 10-fachen molaren Menge, bezogen auf die Verbindung II.

Norbornadien wird zweckmäßigerweise im bis zu 20-fachen Überschuß eingesetzt, üblicherweise 5 bis 15 molar, bezogen auf (II). Das Verfahren wird zwischen 0° und 200° C durchgeführt. Bei der Umsetzung entsteht zunächst das Zwischenprodukt der Formel IV, das bei weiterem Erhitzen zum Endprodukt weiterreagiert. Arbeitet man zunächst bei realtiv niedrigen Temperaturen (0° bis 80° C) so kann bei Zusatz einer löslichen Hilfsbase das Zwischenprodukt der Formel IV isoliert werden.

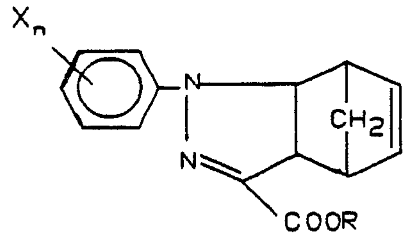

(IV),

Für die nachfolgende Spaltung des Zwischenproduktes IV benötigt man Reaktionstemperaturen von 60° bis 200° C, vorzugsweise 70° bis 160° C.

Die Chlorhydrazone der Formel II sind nach üblichen Verfahren zugänglich. Sie lassen sich beispielsweise aus den entsprechenden Anilinen durch Diazotieren und Kuppeln mit 2-Chlor-Acetessigestern erhalten.

Die bei dieser Synthese erhaltenen Chlorhydrazone können ohne vorherige Reinigung direkt nach Zugabe des Lösungsmittels mit Norbornadien cyclokondensiert werden.

Die folgenden Beispiele dienen der weiteren Erläuterung der Erfindung.

## 1. Herstellungsbeispiele

**Beispiel 1:**

**1-(2,4-Dichlorphenyl)-pyrazol-3-carbonsäureethylester** 14,8 g (0,05 mol) 2-Chlorglyoxylsäureethylester-2,4-dichlorphenylhydrazon (IIa) in 50 ml Toluol werden mit 50 ml Norbornadien und 20 ml Triethylamin 1 h bei 70° C gerührt. Nach Abkühlen wird vom Niederschlag abgesaugt, der Niederschlag mit 20 ml Toluol nachgewaschen und die Toluolphasen im WV (WV = Wasserstrahlvakuum) zur Trockene eingeengt. Der Rückstand wird mit Gradient (Laufmittel: Petrolether (40 bis 70°) bis reiner Essigester) auf Kieselgel chromatographiert. Man erhält 14,5 g (82 % d. Th.) farbloses Öl. Die Identifizierung erfolgt NMR-spektroskopische. 3,5 g (0,01 mol) des so hergestellten 1-(2,4-Dichlorphenyl)-3-ethoxycarbonyl-pyrazolo-[2,2,1]-bicycloheptens werden 3 h im WV auf 150° C erhitzt und nach Abkühlen aus Petrolether umkristallisiert:

Man erhält 2,6 g (88 % d. Th.) Produkt, Nadeln vom Schmp. 88 bis 90° C.

**Beispiel 2:**

**1-(2,4-Dichlorphenyl)-pyrazol-3-carbonsäureethylester**

29,6 g (0,1 mol) 2-Chlor-glyoxylsäureethylester-2,4-dichlorphenylhydrazon (IIa) in 100 ml Toluol werden mit 50 ml Norbornadien und 40 ml Triethylamin 1 h bei 70° C gerührt; nach dem Abkühlen wird der Niederschlag abgesaugt, mit 30 ml Toluol gewaschen und die vereinigten Toluolphasen im WV eingedampft. Der Rückstand wird 3 h im WV auf 160° C erhitzt, nach Abkühlen mit 150 ml Essigsäureethylester/100 ml Wasser aufgenommen, mit 100 ml verdünnter Salzsäure, 100 ml $NaHCO_3$-Lösung und 100 ml Wasser gewaschen, über $MgSO_4$ getrocknet und im WV zur Trockne eingeengt. Nach Säulenchromatographie erhält man 25,6 g (90 % d. Th.) des gewünschten Produkts: Kristalle vom Schmp. 88 bis 90° C.

**Beispiel 3:**

**1-(2,4-Dichlorphenyl)-pyrazol-3-carbonsäureethylester**

14,8 g (IIa) in 100 ml Toluol werden mit 50 ml Norbornadien und 30 ml Pyridin 40 h bei 100° C gerührt. Nach Abkühlen wird im WV zur Trockne eingeengt und wie unter Beispiel 2 beschrieben aufgearbeitet. Man erhält 4,3 g (30 % d. Th.) Kristalle vom Schmp. 88 bis 89° C.

**Beispiel 4:**

**1-(2,4-Dichlorphenyl)-pyrazol-3-carbonsäuremethylester**

14,6 g (0,05 mol) 2-Chlor-glyoxylsäuremethylester-2,4-dichlorphenylhydrazon in 100 ml Toluol werden mit 50 ml Norbornadien und 10 g $NaHCO_3$ 24 h bei 70° C gerührt. Nach Abkühlen wird wie unter Beispiel 2 beschrieben aufgearbeitet: Man erhält 6,2 g (46 % d. Th.) Kristalle vom Schmp. 105 bis 107° C.

**Beispiel 5**

**1-(2,4-Dichlorphenyl)-pyrazol-3-carbonsäureethylester**

5,9 g (0,020 mol) IIa in 50 ml DMF werden mit 20 ml Norbornadien und 5 g $NaHCO_3$ 2 h bei RT gerührt; der erhaltene Niederschlag wird abgesaugt und mit 20 ml DMF nachgewaschen. Das Filtrat wird im WV zur Trockne eingeengt und der erhaltene Rückstand 3 h auf 160° C erhitzt. Nach Säulenchromatographie (Petrolether (30 bis 70°) bis Essigester auf Kieselgel) erhält man 4,6 g (81 % d. Th.) Kristalle vom Schmp. 88 bis 90° C. DMF= Dimethylformamid

**Beispiel 6:**

**1-(2,4-Dichlorphenyl)-pyrazol-3-carbonsäureethylester**

5,9 g (0,02 mol) IIa in 50 ml DMF werden bei 100° C mit 20 ml Norbornadien und 6 g $K_2CO_3$ 8 h gerührt. Nach Abkühlen wird vom Niederschlag abgesaugt, dieser wird mit 20 ml DMF nachgewaschen. Das Filtrat wird im WV zur Trockne eingeengt und wie in Beispiel 5 beschrieben, chromatographiert. Man erhält 2,1 g (37 % d. Th.) Kristalle vom Schmp. 88 bis 90° C.

**Beispiel 7:**

**1-(2,4-Dichlorphenyl)-pyrazol-3-carbonsäureethylester**

5,9 g (0,02 mol) IIa werden in 50 ml DMF mit 20 ml Norbornadien 40 h bei 140° C gerührt. Man engt im WV zur Trockne ein und chromatographiert über Kieselgel. Man erhält 1,9 g (33 % d. Th.) Kristalle, Schmp. 88 bis 90° C.

**Beispiel 8:**

**1-(2,4-Dichlorphenyl)-pyrazol-3-carbonsäureethylester**

5,9 g (0,02 mol) IIa in 40 ml DMSO werden mit 20 ml Norbornadien und 6 g $KHCO_3$ 3 h bei RT gerührt und dann 3 h bei 150° C gerührt. Man gießt auf 100 ml Wasser und saugt vom Niederschlag ab. Nach Säulenchromatographie erhält man 4,7 g (82 % d. Th.) Kristalle vom Schmp. 88 bis 90° C.

Analog lassen sich folgende Ester der Formel I synthetisieren.

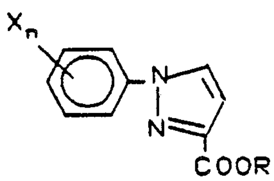

(I)

| Vbg. Bsp.-Nr. | $X_n$ | R | Schmp. °C | Ausb. % | analog Bsp.-Nr. |
|---|---|---|---|---|---|
| 9 | 2,4-$Br_2$ | $C_2H_5$ | 103-5 | 68 | 1 |
| 10 | 4-O-n-$C_4H_9$-2-$CF_3$ | $C_2H_5$ | Oel | 78 | 2 |
| 11 | 4-$CH_3O$ | $C_2H_5$ | Oel | 71 | 1 |
| 12 | 3-Cl-2,6-$(C_2H_5)_2$ | $C_2H_5$ | Oel | 64 | 1 |
| 13 | 4-F-3-$CF_3$ | $C_2H_5$ | 24-29 | 73 | 5 |
| 14 | 2-Cl-4-$CF_3$ | $C_2H_5$ | 38-41 | 78 | 8 |
| 15 | 2,4-$F_2$-3,5-$Cl_2$ | $C_2H_5$ | Oel | 61 | 1 |
| 16 | 4-Cl-2-F-5-$OCH_3$ | $C_2H_5$ | 90-92 | 79 | 2 |
| 17 | 2,6-$(C_2H_5)_2$ | $C_2H_5$ | Oel | 72 | 2 |

**Beispiel 18:**

**1-(2,4-Dichlorphenyl)-pyrazol-3-carbonsäure**

5,9 g (0,02 mol) IIa in 100 ml Toluol werden unter Kühlung mit 20 ml Norbornadien und 10 g KOH-Pulver versetzt und 24 h bei RT gerührt. Man wäscht die organische Phase mit 50 ml verdünnter Salzsäure, trocknet sie und engt sie im WV zur Trockne ein. Das erhaltene zähe Oel wird 3 h im WV auf 160° C erhitzt und nach Erkalten aus Ethanol umkristallisiert. Man erhält 3,2 g (62 % d. Th.) Kristalle vom Schmp. 177 bis 180° C.

## Ansprüche

1. Verfahren zur Herstellung von Verbindungen der Formel I

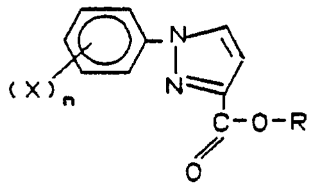

(I),

worin
X unabhängig voneinander Halogen, ($C_1$-$C_6$)-Alkyl, ($C_1$-$C_6$)-Alkoxy, Halogen($C_1$-$C_6$)alkyl oder Halogen-($C_1$-$C_6$)alkoxy,
R H, ($C_1$-$C_6$)-Alkyl und
n eine ganze Zahl von 1 bis 5 bedeuten,
dadurch gekennzeichnet, daß man ein Chlorhydrazon der Formel II

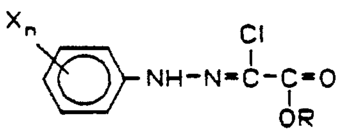

(II)

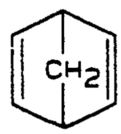

(III)

mit Norbornadien der Formel III umsetzt.

2. Verfahren gemäß Anspruch 1, dadurch gekennzeichnet, daß in Formel II R = ($C_1$-$C_4$)-Alkyl, X = Halogen, $C_1$-Halogenalkyl oder $C_1$-Halogenalkoxy und n = 1, 2 oder 3 bedeutet.

3. Verfahren gemäß Anspruch 1 oder 2, dadurch gekennzeichnet, daß man die Umsetzung in einem inerten organischen Lösungsmittel durchführt.

4. Verfahren gemäß einem der Ansprüche 1 bis 3, dadurch gekennzeichnet, daß man die Umsetzung in Gegenwart einer organischen oder anorganischen Hilfsbase durchführt.

5. Verfahren gemäß einem oder mehreren der Ansprüche 1 bis 4, dadurch gekennzeichnet, daß man die Umsetzung bei Temperaturen zwischen 0° und 200° C durchführt.

6. Verfahren gemäß einem oder mehreren der Ansprüche 1 bis 4, dadurch gekennzeichnet, daß man das Verfahren zunächst zwischen 0° und 80° C und anschließend zwischen 80° C und 160° C durchführt.

7. Verfahren gemäß Anspruch 4, 5 oder 6, dadurch gekennzeichnet, daß man die Hilfsbase in der 1- bis 10-fachen molaren Menge bezogen auf die Komponente II einsetzt.

8. Verfahren gemäß einem oder mehreren der Ansprüche 1 bis 7, dadurch gekennzeichnet, daß man Norbornadien in 1-bis 20-facher Mol-Menge bezogen auf die Komponente II einsetzt.

Europäisches Patentamt

# EUROPÄISCHER RECHERCHENBERICHT

Nummer der Anmeldung

EP 88 11 3834

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | CHEMISCHE BERICHTE, Band 101, Nr. 2, 1. Februar 1968, Seiten 536-551, Verlag Chemie GmbH, Weinheim, DE; R. HUISGEN et al.: "Pyrazole aus Sydnonen und acetylenischen Dipolarophilen" * Seiten 537-538,543-545 * --- | | C 07 D 231/14 |
| D,P A | EP-A-0 269 806 (HOECHST) ----- | | |
| | | | RECHERCHIERTE SACHGEBIETE (Int. Cl.4) |
| | | | C 07 D 231/00 |

Der vorliegende Recherchenbericht wurde für alle Patentansprüche erstellt

| Recherchenort | Abschlußdatum der Recherche | Prüfer |
|---|---|---|
| DEN HAAG | 05-12-1988 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)